(19) Europäisches Patentamt
European Patent Office
Office européen des brevets

(11) Veröffentlichungsnummer: **0 009 239**
**B1**

(12) # EUROPÄISCHE PATENTSCHRIFT

(45) Veröffentlichungstag der Patentschrift: **15.07.81**

(21) Anmeldenummer: **79103516.5**

(22) Anmeldetag: **19.09.79**

(51) Int. Cl.³: **C 07 C 45/36,** C 07 C 47/542, C 07 C 47/575 // B01J23/88, B01J23/28, B01J23/30, B01J23/32, B01J23/64

(54) **Verfahren zur Herstellung von p-substituierten Benzaldehyden.**

(30) Priorität: **25.09.78 DE 2841712**

(43) Veröffentlichungstag der Anmeldung: **02.04.80 Patentblatt 80/7**

(45) Bekanntmachung des Hinweises auf die Patenterteilung: **15.07.81 Patentblatt 81/28**

(84) Benannte Vertragsstaaten: **CH DE FR GB NL**

(56) Entgegenhaltungen:
**FR-A-2 279 465**
**FR-A-2 292 518**
**FR-A-2 357 512**

(73) Patentinhaber: **BASF Aktiengesellschaft, Carl-Bosch-Strasse 38, D-6700 Ludwigshafen (DE)**

(72) Erfinder: **Krabetz, Richard, Dr. Dipl.-Chem., Unterer Waldweg 8, D-6719 Kirchheim (DE)**
Erfinder: **Siegel, Hardo, Dr. Dipl.-Chem., Hans-Purrmann-Allee 25, D-6720 Speyer (DE)**

EP 0 009 239 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

# Verfahren zur Herstellung von p-substituierten Benzaldehyden

Die Erfindung betrifft ein Verfahren zur Herstellung von p-substituierten Benzaldehyden durch Dampfphasenoxidation von p-substituierten Alkylbenzolen in Gegenwart von molybdänhaltigen Katalysatoren.

Molybdän enthaltende Katalysatoren sind schon für die Gasphasenoxidation von Alkylaromaten herangezogen worden. So wird in der Be-PS 786551 ein Verfahren zur Herstellung von Benzaldehyd beschrieben, bei dem man die Gasphasenoxidation von Toluol mit Katalysatoren durchführt, die neben Mo mindestens eines der Elemente Fe, Ni, Co, Sb, Bi, V, P, Sm, Ta, Sn oder Cr enthalten. Diese bekannten Katalysatoren sind für die Oxidation von Alkylaromaten zu Benzaldehyden, die in p-Stellung zu der zu oxidierenden Methylgruppe eine Alkyl- oder Methoxygruppe enthalten, wegen mangelhafter Selektivität wenig geeignet.

Den gleichen Mangel weisen die Katalysatorengemische aus den Oxiden des Wolframs und Molybdäns auf, die man gemäss der DT-AS 1947994 für die Oxidation von Toluol oder o-Xylol zu den entsprechenden Aldehyden verwendet. Ihre mangelhafte Selektivität führt z.B. bei der Oxidation von p-Xylol zu guten Ausbeuten an Terephthaldehyd, jedoch zu nur geringen Ausbeuten an Tolualdehyd.

Aus der FR-PS 2279465 sind Oxidationskatalysatoren, die Molybdänoxid neben anderen Metalloxiden enthalten, bekannt, welche für die Oxidation von Olefinen geeignet sind. Es ist auch angegeben, das man mit diesen Katalysatoren Aromaten mit einer Methylgruppe oxidieren kann. Dass sich Toluole mit p-ständigen Substituenten selektiv an der Methylgruppe oxidieren lassen, kann dieser Patentschrift jedoch nicht entnommen werden.

In der FR-PS 2357512 und in der FR-PS 2292518 wird die Herstellung von α, β-ungesättigten Aldehyden beschrieben, bei der man Olefine in Gegenwart von Katalysatoren, die Oxide des Kobalts, Eisens, Nickels sowie andere Metalloxide enthalten, oxidiert. Dass diese Katalysatoren für die selektive Oxidation von p-substituierten Toluolen geeignet sind, kann dieser Literatur nicht entnommen werden.

Es wurde nun gefunden, dass man p-substituierte Benzaldehyde der Formel

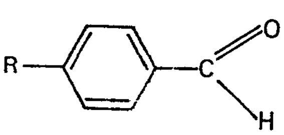

I,

in der R eine Methyl-, Methoxy- oder eine tertiäre Butylgruppe bedeutet, durch Dampfphasenoxidation eines Alkylbenzols der Formel

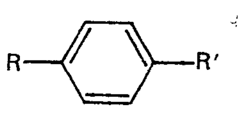

II,

in der R′ für einen Alkylrest steht, mit Sauerstoff oder Sauerstoff enthaltenden Gasen in Gegenwart von molybdänhaltigen Katalysatoren bei Temperaturen von 150 bis 600 °C vorteilhaft herstellen kann, wenn man einen Metalloxidkatalysator verwendet, der eine Zusammensetzung entsprechend der allgemeinen Formel aufweist:

$$Mo_{12}\,Me^1_a\,Me^2_b\,Me^3_c\,Me^4_d\,O_x \qquad \text{III,}$$

worin stehen:

$Me^1$ für eines oder mehrere der Elemente Bi, Co, Ni, Fe, W, Nb, Ta,

$Me^2$ für eines oder mehrere der Elemente K, Rb, Cs, Tl, In,

$Me^3$ für eines oder mehrere der Elemente P, B, Sb, V, Sr, Cr,

$Me^4$ für eines oder mehrere der Elemente Mn, Re, Pd, Ir, Rh, Cu, Sn, Zn, Sm, Mg, Ce, Ag, Li, As, Ba, Ca.

a für eine Zahl von 0 bis 24, jedoch für den Fall, dass W enthalten ist, für eine Zahl von 0,5 bis 110,

b für eine Zahl von 0,01 bis 0,2, jedoch für den Fall, dass von Bi, V oder P mehr als 0,5 Atome vorhanden sind, für eine Zahl von 0,1 bis 6,

c für eine Zahl von 0 bis 6, jedoch für den Fall, dass a für 0 steht, für eine Zahl von 0,5 bis 5,

d für eine Zahl von 0 bis 10, und

x für die Zahl, die sich formal aus der Summe der vorhandenen Metall- und Phosphoratome und deren höchste Oxidationsstufe ergibt.

Von den Katalysatoren der Formel III sind solche bevorzugt, die als $Me^2$ In zusammen mit einem oder mehreren der Elemente K, Rb, Cs und Tl enthalten.

Das erfindungsgemässe Verfahren wird bei Temperaturen von 150 bis 600 °C, vorzugsweise 200 bis 460 °C und zweckmässig bei Verweilzeiten von 0,01 bis 10 Sekunden durchgeführt. Als Oxidationsmittel verwendet man Sauerstoff oder vorzugsweise Luft. Vorteilhaft verdünnt man das Gemisch aus dem Alkylbenzol und Sauerstoff bzw. Luft mit inerten Verdünnungsmitteln, wie Wasserdampf, Kohlendioxid und Stickstoff. Das der Oxidation zugeführte Ausgangsgemisch kann in weiten Grenzen variiert werden. Im allgemeinen weist das Gemisch einen Gehalt an dem zu oxidierenden Alkylbenzol von 1 bis 20, vorzugsweise 2 bis 10 Volumenprozent auf. Das Volumenverhältnis Sauerstoff zu Alkylbenzol beträgt vorteilhaft 0,5 bis 4 zu 1, insbesondere 1 bis 2,5 zu 1. Wird nur mit Luft als Oxidationsmittel ohne inerte Verdünnungsmittel gearbeitet, so beträgt das Volumenverhältnis Alkylbenzol zu Luft zweckmässig 1:30 bis 1:100.

Mit den Katalysatoren der allgemeinen Formel III werden die p-substituierten Alkylbenzole in hoher Selektivität, d.h. unter weitgehender Aufrechterhaltung des para-ständigen Substituenten, in die gewünschten p-substituierten Benzaldehyde übergeführt. Von besonderem technischem Interesse ist die Herstellung von p-tert.-

Butylbenzaldehyd aus p-tert.-Butyltoluol, wobei man als wertvolles Nebenprodukt die p-tert.-Butylbenzoesäure erhält. Das p-tert.-Butylbenzaldehyd selbst ist ein wertvolles Zwischenprodukt für die Herstellung von Riechstoffen.

Besonders vorteilhafte Ergebnisse beim erfindungsgemässen Verfahren, insbesondere bei der Herstellung von p-tert.-Butylbenzaldehyd, werden erhalten, wenn man Katalysatoren verwendet, die eine Zusammensetzung der folgenden allgemeinen Formel IV aufweisen:

$$Mo_{12}\ Fe_{0,5-6}\ Me^5_e\ Me^6_f\ Me^7_g\ Me^4_h\ Bi_i\ O_x \qquad IV$$

worin stehen:
$Me^5$ für Ni und oder Co,
$Me^6$ für eines oder mehrere der Elemente P, B, Sb,
$Me^7$ für eines oder mehrere der Elemente K, Rb, Cs, Tl, In,
e für eine Zahl von 0 bis 12,
f für eine Zahl von 0 bis 6,
g für eine Zahl von 0,1 bis 0,2,
h für eine Zahl von 0 bis 3,
i für eine Zahl von 0 bis 6
und $Me^4$ und x die oben genannte Bedeutung haben.

Von den Katalysatoren der Formel IV sind solche bevorzugt, die als $Me^7$ In zusammen mit einem oder mehreren der Elemente K, Rb, Cs und Tl enthalten, und in denen $Me^6$ B und/oder Sb, $Me^4$ Zn und/oder Mg, e eine Zahl von 0,3 bis 10, f eine Zahl von 0,05 bis 3, i eine Zahl von 0,1 bis 6 und h eine Zahl von 0,01 bis 3 bedeuten. Diese Katalysatoren entfalten ihre optimalen Ergebnisse bei Temperaturen von 200 bis 400 °C.

Von besonderem technischen Interesse sind auch die Katalysatoren der Formel V, insbesondere für die Herstellung von p-Toluylaldehyd aus p-Xylol. Diese Katalysatoren haben die folgende Zusammensetzung:

$$Mo_{12}\ W_{0,5-100}\ Me^8_k\ Me^9_l,\ Me^{10}_m\ O_x \qquad V$$

worin stehen:
$Me^8$ für eines oder mehrere der Elemente Bi, Fe, Ce, Mn, Cu, Zn
$Me^9$ für eines oder mehrere der Elemente Sb, Cr, V, B
$Me^{10}$ für eines oder mehrere der Elemente K, Rb, Cs, Tl, In.
k für eine Zahl von 0 bis 10,
l für eine Zahl von 0 bis 6,
m für eine Zahl von 0,01 bis 1
und x die oben genannte Bedeutung hat. Von den Katalysatoren der Formel V sind solche bevorzugt, in denen k eine Zahl von 0,1 bis 3, l eine Zahl von 0,01 bis 3 und m eine Zahl von 0,01 bis 0,1 bedeuten.

Die Katalysatoren können mit einem inerten Trägermaterial gemischt oder auf inerte, geformte Träger aufgebracht sein. Als Trägermaterialien kommen die Oxide, Hydroxide oder Sauerstoffsäuren der Elemente Ti, Si, Al und Mg in Betracht, ferner Erdalkalisilikate, Aluminiumsilikate oder Siliziumkarbid. Die Trägermenge macht zwischen 5 und 95, vorzugsweise 8 bis 90 Gewichtsprozent des fertigen Katalysators aus. Als geformte Träger eignen sich Kugeln oder Ringe aus keramischen Massen wie $\alpha$-$Al_2O_3$, Mullit, Steatit, Siliziumkarbid mit inneren Oberflächen unter 100, vorzugsweise unter 10 $m^2/g$.

Die Katalysatoren werden vorteilhaft als Schalen- oder Schicht-Katalysatoren eingesetzt, in denen die Oxide der aktiven Komponenten in dünner Schicht von 10 bis 3000 µm auf die äussere Oberfläche von inerten oberflächenarmen geformten Trägern aufgebracht sind. Als Träger eignen sich insbesondere Kugeln oder Ringe aus $Al_2O_3$, Erdalkali- und Aluminiumsilikaten oder $SiO_2$.

Die Katalysatoren können sowohl im Festbett als auch im Wirbelbett eingesetzt werden. Um hohe Selektivitäten des Aldehyds zu erreichen, ist es zweckmässig, Verweilzeit und Temperatur so zu wählen, dass der Umsatz der Alkylbenzolverbindung unter 80 Mol-%, vorzugsweise unter 60 Mol-%, insbesondere unter 40% liegt. Das Abgas aus dem Katalysator kann gegebenenfalls nach weitgehender Abtrennung des Aldehyds, z.B. durch Kondensation und Gaswäsche, zum Teil in die Synthese als Verdünnungsmittel für das aus Alkylbenzolderivat und Sauerstoff bzw. Luft bestehende Gemisch zurückgeführt werden. Die kondensierten Anteile werden nach Abtrennung der wässrigen Schicht destillativ aufgetrennt; das nicht umgesetzte Substrat wird wieder in die Synthese zurückgeführt.

Im folgenden bedeuten:

$$\text{Umsatz} = \frac{\text{umgesetzte Ausgangsverbindung} \times 100}{\text{eingesetzte Ausgangsverbindung}}\ [\text{mol/mol}]$$

$$\text{Ausbeute} = \frac{\text{gebildeter Aldehyd} \times 100}{\text{eingesetzte Ausgangsverbindung}}\ [\text{mol/mol}]$$

$$\text{Selektivität} = \frac{\text{Ausbeute} \times 100}{\text{Umsatz}}\ [\text{mol/mol}]$$

Beispiel 1

Es werden folgende Lösungen bzw. Salze bereitgestellt:

A 212 g Ammoniumheptamolybdat $(NH_4)_6Mo_7O_{24} \cdot 4\ H_2O$ in 1200 g destilliertem Wasser
B 1,4 g Kaliumnitrat und 0,22 g Indiumchlorid
C 204 g Kobaltnitrat $Co(NO_3)_2 \cdot 6\ H_2O$ in 100 ml Wasser
D 121 g Eisen(III)nitrat $Fe(NO_3)_3 \cdot 9\ H_2O$ in 100 ml

Wasser

E 48,5 g Wismutnitrat $Bi(NO_3)_3 \cdot 5\ H_2O$ gelöst in einer Mischung aus 120 ml Wasser und 16 ml konz. Salpetersäure

F 12,4 g Borsäure $H_3BO_3$ in 25 ml Wasser

G 29 g Nickelnitrat $Ni(NO_3)_2 \cdot 6\ H_2O$ in 25 ml Wasser

H 2,9 g Antimontrioxid $Sb_2O_3$

I 98 g $SiO_2$ (Aerosil)

Die Lösung A wird vorgelegt, dann werden die Lösungen bzw. Salze B bis I unter Rühren zugegeben. Die erhaltene Aufschlämmung wird auf dem Wasserbad eingedickt und bei 10 °C getrocknet. Die Trockenmasse wird auf eine Körnung von 2 bis 4 mm zerkleinert und 6 Stunden bei 650 °C calciniert.

Die formale Zusammensetzung des Katalysators entspricht der Formel (ohne $SiO_2$)

$$Mo_{12}Co_7Ni_1Fe_3Bi_1Sb_{0,1}B_2K_{0,14}In_{0,01}\ O_{56,7}$$

40 ml Katalysator werden in ein U-förmiges Stahlrohr mit einem Innendurchmesser von 15 mm eingefüllt. Das Rohr befindet sich in einem elektrisch beheizten, gerührten Bad aus geschmolzenen Salzen. Durch das Rohr werden stündlich ein Gemisch aus 1,5 Nl p-tert.-Butyl-toluol, 20 Nl Luft und 18,6 Nl Wasserdampf bei einer Badtemperatur von 365 °C geleitet. Nach der Analyse der Austräge beträgt der Umsatz 19,8 Mol.-%, die Ausbeute an p-tert.-Butyl-benzaldehyd 9 Mol.-% und die Ausbeute an p-tert.-Butylbenzoesäure 8,5 Mol.-%. Die Selektivität hinsichtlich der zwei Produkte beträgt 45,5 Mol.-% bzw. 43 Mol.-%. Der Versuch wird wiederholt mit einer Katalysatormenge von 20 ml. Bei einer Badtemperatur von 380 °C werden 14,8% des p-tert.-Butyltoluols umgesetzt. Bei Einsatz von 10 ml Katalysator wird bei 394 °C ein Umsatz von 10% und eine Selektivität der Aldehydbildung von 62 Mol.-% erreicht.

Beispiel 2 bis 8

Nach der Vorschrift des Beispiels 1 werden verschiedene Katalysatoren hergestellt, in denen einzelne Komponenten weggelassen oder durch andere Komponenten ersetzt sind, oder in denen die Mengenverhältnisse der Komponenten geändert wurden.

In Tabelle 1 sind die formale Zusammensetzung der Katalysatoren und die Ergebnisse der Aktivitätsprüfung zusammengefasst.

Die bisher nicht genannten Komponenten wurden in folgender Form eingesetzt:

$Nb_2O_5$, $Ta_2O_5$, $RbNO_3$, $CsNO_3$, $NH_4VO_3$,$(NH_4)_{10}W_{12}O_{41}$, $H_3PO_4$, $TlNO_3$, $Mg(NO_3)_2 \cdot 4\ H_2O$, $In(NO_3)_3$, $SnO_2$,$Zn(NO_3)_2 \cdot 6\ H_2O$, $Sr(NO_3)_2 \cdot H_2O$, $Ba(NO_3)_2$, $Ce(NO_3)_3 \cdot 6\ H_2O$, $Cr(NO_3)_3 \cdot 9\ H_2O$,$UO_2(NO_3)_2 \cdot 6\ H_2O$,$TiO_2$.

Tabelle 1

| Beispiel | Katalysatorzusammensetzung (ohne Oxidsauerstoff) | Calcinierungstemp. und Zeit Kat.-Menge | Badtemp. °C | Umsatz Mol.-% | Ausbeute Aldehyd | Mol.-% Säure | Selektivität Mol.-% Aldehyd |
|---|---|---|---|---|---|---|---|
| 2 | $Mo_{12}\ Bi_1\ Ni_6\ Co_{0,3}Fe_3Sb_{0,1}\ Zn_2B_2Rb_{0,14}In_{0,01}$ | 650 °C/6 h 40 ml | 365 | 21 | 9 | 9 | 43 |
| 3 | $Mo_{12}Bi_1Ni_8Co_{0,3}\ Fe_3Sb_{0,1}B_2In_{0,2}K_{0,14}$ | 650 °C/6 h 40 ml | 366 | 22 | 7 | 8 | 32 |
| 4 | $Mo_{12}Ni_{8,5}Fe_2Bi_1\ Zn_2P_{0,06}In_{0,1}K_{0,06}$ | 580 °C/1,5 h 20 ml | 362 | 24 | 9 | 10,3 | 37,5 |
| 4a | $Mo_{12}Bi_9Nb_1K_{0,02}$ | 500 °C/3 h 40 ml | 400 | 25 | 6 | 1,5 | 24 |
| 5 | $Mo_{12}P_2V_1Cs_2\ Sr_{0,5}$ | 480 °C/16h 40 ml | 420 | 19 | 3 | 1,5 | 16 |
| 6 | $Mo_{12}P_1V_1Cs_1\ Sn_1$ | 420 °C/4h 40 ml | 400 | 13 | 5 | 2 | 39 |
| 7 | $Mo_{12}P_2Ta_{4,4}\ K_{4,8}$ | 450 °C/16 h 40 ml | 375 | 15 | 3,5 | 0,5 | 23 |
| 8 | $Mo_{12}Bi_1Fe_1Ni_4\ Co_{4,5}P_1Mg_1\ Tl_{0,1}In_{0,01}$ | 530 °C/6 h 30 ml | 365 | 18 | 8 | 7 | 44 |

Beispiel 9

Ein auf einen $Al_2O_3$-Träger aufgebrachter Katalysator der Zusammensetzung $Mo_{12}\ V_3\ W_{1,2}\ Cu_{1,u}\ K_{0,01}\ O_x$ wird wie folgt hergestellt:

212 g Ammoniumheptamolybdat

35 g Ammoniummetavanadat

32,3 g Ammoniumparawolframat

32,3 g basisches Kupferacetat (MG = 369,27)

0,056 g Kaliumhydroxid

werden in der genannten Reihenfolge in 1500 g siedendes Wasser eingetragen. Die Suspension wird auf dem Wasserbad bei 90 bis 100 °C eingetrocknet und bei 110 °C nachgetrocknet. Die Masse wird nach Zugabe von 20 Gewichtsprozent Wasser im Kneter 3½ Stunden verknetet und nochmals bei 140 °C getrocknet. Anschliessend wird die Masse bei ansteigenden Temperaturen von 210 bis 390 °C 4 Stunden und dann 3 Stunden bei 400 °C in Luft calciniert. Die calcinierte Masse wird auf eine Körnung kleiner als 60 μm zerkleinert.

Das Katalysatorpulver wird auf $Al_2O_3$-Trägerkugeln mit einem Durchmesser von 3 mm in einer Menge von 34 Gewichtsprozent in Gegenwart von Wasser aufgebracht. Nach Trocknung bei 140 °C ist der Katalysator einsatzfähig.

40 ml Katalysator werden in ein Stahlrohr von 11 mm innerem Durchmesser eingefüllt. Über den Katalysator wird stündlich ein Gemisch aus 1,5 Nl p-tert.-Butyl-toluol, 20 Nl Luft und 20 Nl Stickstoff bei einer Salzbadtemperatur von 330 °C geleitet. Aus der Analyse der Austräge errechnet sich ein Umsatz von 15 Mol-% und eine Ausbeute an Aldehyd von 6,9 Mol.-%. Die Selektivität bezüglich der Umsetzung des p-tert.-Butyl-toluols zu Aldehyd beträgt 46.

Beispiel 10 bis 17

Entsprechend der vorangegangenen Beispiele werden weitere Katalysatoren hergestellt mit dem Unterschied, dass das Kupfersalz durch Manganacetat-tetrahydrat, Eisen(II)-oxalat, Cernitrat, Zinknitrat, Wismutnitrat, Antimon(III)-oxid ersetzt wird und dass gegebenenfalls in der wässrigen Lösung des Molybdats konz. Phosphorsäure oder Borsäure gelöst wird. Die Ergebnisse sind in Tabelle 2 zusamengefasst.

Die Prüfung der katalytischen Wirksamkeit erfolgte in den Beispielen 10 bis 17 mit einem Gasgemisch aus stündlich 2,6 Nl tert.-Butyl-toluol, 20 Nl Luft und 15 Nl Wasserdampf.

Tabelle 2

| Beispiel | Katalysatorzusammensetzung (ohne Oxidsauerstoff) | Calcinierungstemp. und Zeit Kat.-Menge | Badtemp. °C | Umsatz Mol.-% | Ausbeute Mol.-% Aldehyd | Ausbeute Mol.-% Säure | Selektivität Mol.-% Aldehyd |
|---|---|---|---|---|---|---|---|
| 10 | $Mo_{12}W_{1,2}V_3Fe_{1,8}Rb_{0,03}$ | 400 °C/5 h 20 ml | 360 | 13 | 4,4 | 3,3 | 34 |
| 11 | $Mo_{12}W_{1,2}V_3Ce_{1,8}K_{0,04}$ | 400 °C/5 h 20 ml | 370 | 14 | 5,5 | 3 | 39 |
| 12 | $Mo_{12}W_{1,2}V_3Mn_{1,8}Cs_{0,04}$ | 400 °C/5 h 20 ml | 380 | 15 | 5,6 | 3 | 37 |
| 13 | $Mo_{12}W_{1,2}V_3$ | 415 °C/1 h 20 ml | 340 | 11,2 | 3,2 | 3,4 | 29 |
| 14 | $Mo_{12}W_3V_2B_3SbK_{0,03}$ | 415 °C/1 h 20 ml | 355 | 10,2 | 6,1 | 3 | 60 |
| 15 | $Mo_1W_1V_1Zn_3Bi_3K_{0,02}$ | 550 °C/16h 30 ml | 400 | 26,8 | 5,9 | 1 | 22 |
| 16 | $Mo_{12}W_{1,2}V_3Cr_2K_{0,06}$ | 400 °C/5 h 20 ml | 390 | 18 | 7 | 3 | 39 |
| 17*) | $Mo_{12}Fe_6K_{0,03}$ | 400 °C/4h 30 ml | 320 | 15 | 4,3 | 1,7 | 29 |

*) ohne Träger

Beispiel 18

Über 20 ml des nach Beispiel 1 hergestellten Katalysators wird ein Gemisch aus 1,5 Nl p-Methoxy-toluol (= 4-Anisol), 20 Nl Luft und 18,6 Nl Wasserdampf bei einer Badtemperatur von 370 °C geleitet. Nach der Analyse der Austräge beträgt der Umsatz 10 Mol.-%, und die Ausbeute an p-Methoxy-benzaldehyd 5 Mol.-% bzw. die Selektivität 50 Mol.-%.

Beispiel 19

Über 20 ml des nach Beispiel 1 hergestellten Katalysators wird stündlich ein Gemisch aus 1,95 Nl p-Xylol und 100 Nl Luft bei einer Badtemperatur von 394 °C geleitet. Nach der Analyse der Austräge beträgt der Umsatz 17,1 Mol.-%, die Ausbeute von α-Toluylaldehyd 10,3 Mol.-% und die Selektivität 60,2 Mol.-%. Terephthalaldehyd wird mit einer Selektivität von 9% gebildet.

**Patentansprüche**

1. Verfahren zur Herstellung von p-substituierten Benzaldehyden der Formel

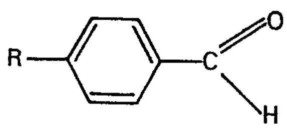

I,

in der R eine Methyl-, Methoxy- oder eine tertiäre Butylgruppe bedeutet, durch Dampfphasenoxidation eines Alkylbenzols der Formel

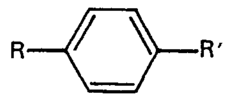

II,

in der R′ für einen Alkylrest steht, mit Sauerstoff oder Sauerstoff enthaltenden Gasen in Gegenwart von molybdänhaltigen Katalysatoren bei Temperaturen von 150 bis 600°C, dadurch gekennzeichnet, dass man einen Metalloxidkatalysator verwendet, der eine Zusammensetzung entsprechend der allgemeinen Formel aufweist:

$$Mo_{12}\, Me^1_a\, Me^2_b\, Me^3_c\, Me^4_d O_x \qquad III$$

worin stehen:
$Me^1$ für eines oder mehrere der Elemente Bi, Co, Ni, Fe, W, Nb, Ta,
$Me^2$ für eines oder mehrere der Elemente K, Rb, Cs, Tl, In,
$Me^3$ für eines oder mehrere der Elemente P, B, Sb, V, Sr, Cr,
$Me^4$ für eines oder mehrere der Elemente Mn, Re, Pd, Ir, Rh, Cu, Sn, Zn, Sm, Mg, Ce, Ag, Li, As, Ba, Ca,
a für eine Zahl von 0 bis 24, jedoch für den Fall, dass W enthalten ist, für eine Zahl von 0,5 bis 110,
b für eine Zahl von 0,01 bis 0,2, jedoch für den Fal, dass von Bi, V oder P mehr als 0,5 Atome vorhanden sind, für eine Zahl von 0,01 bis 6,
c für eine Zahl von 0 bis 6, jedoch für den Fall, dass a für 0 steht, für eine Zahl von 0,5 bis 5,
d für eine Zahl von 0 bis 10, und
x für die Zahl, die sich formal aus der Summe der vorhandenen Metall- und Phosphoratome und deren höchste Oxidationsstufe ergibt.

2. Verfahren für die Herstellung von p-tert.-Butylbenzaldehyd aus p-tertiär-Butyltoluol nach Anspruch 1, dadurch gekennzeichnet, dass man einen Metalloxidkatalysator verwendet, der eine Zusammensetzung entsprechend der allgemeinen Formel aufweist:

$$Mo_{12}\, Fe_{0,5\text{-}6}\, Me^5_e\, Me^6_f\, Me^7_g\, Me^4_h\, Bi_i\, O_x \qquad IV$$

worin stehen:
$Me^5$ für Ni und/oder Co,
$Me^6$ für eines oder mehrere der Elemente P, B, Sb,
$Me^7$ für eines oder mehrere der Elemente K, Rb, Cs, Tl, In,
e für eine Zahl von 0 bis 12,
f für eine Zahl von 0 bis 6,
g für eine Zahl von 0,01 bis 0,2,
h für eine Zahl von 0 bis 3,
i für eine Zahl von 0 bis 6
und $Me^4$ und x die in Anspruch 1 genannte Bedeutung haben.

3. Verfahren für die Herstellung von p-Toluylaldehyd aus p-Xylol nach Anspruch 1, dadurch gekennzeichnet, dass man einen Metalloxidkatalysator verwendet, der die Zusammensetzung entsprechend der allgemeinen Formel aufweist:

$$Mo_{12}\, W_{0,5\text{-}100}\, Me^8_k\, Me^9_l,\, Me^{10}_m\, O_x \qquad V$$

worin stehen:
$Me^8$ für eines oder mehrere der Elemente Bi, Fe, Ce, Mn, Cu, Zn,
$Me^9$ für eines oder mehrere der Elemente Sb, Cr, V, B,
$Me^{10}$ für eines oder mehrere der Elemente K, Rb, Cs, Tl, In,
k für eine Zahl von 0 bis 10,
l für eine Zahl von 0 bis 6,
m für eine Zahl von 0,01 bis 1,
und in der x die in Anspruch 1 genannte Bedeutung hat.

**Claims**

1. A process for the preparation of a p-substituted benzaldehyde of the formula

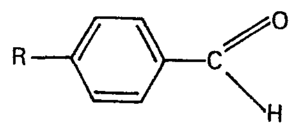

I,

where R is methyl, methoxy or tertiary butyl, by vapor phase oxidation of an alkylbenzene of the formula

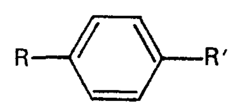

II,

where R′ is alkyl, with oxygen or an oxygen-containing gas in the presence of a molybdenum-containing catalyst at temperatures of from 150° to 600°C, characterized in that a metal oxide catalyst is used which has a composition corresponding to the general formula

$$Mo_{12}\, Me^1_a\, Me^2_b\, Me^3_c\, Me^4_d\, O_x \qquad III$$

where
$Me^1$ is one or more of the elements Bi, Co, Ni, Fe, W, Nb and Ta,
$Me^2$ is one or more of the elements K, Rb, Cs, Tl and In,
$Me^3$ is one or more of the elements P, B, Sb, V, Sr and Cr,
$Me^4$ is one or more of the elements Mn, Re, Pd, Ir, Rh, Cu, Sn, Zn, Sm, Mg, Ce, Ag, Li, As, Ba and Ca,
a is a number from 0 to 24, but if W is present, is a number from 0.5 to 110,
b is a number from 0.01 to 0.2, but if more than 0.5 atom of Bi, V or P is present, is a number from 0.01 to 6,
c is a number from 0 to 6, but if a is 0, is a number

from 0.5 to 5,
d is a number from 0 to 10, and
x is the number which results formally from the sum of metal atoms and phosphorus atoms present and their highest oxidation state.

2. A process for the preparation of a o-tert.-butylbenzaldehyde from p-tert.-butyltoluene as claimed in claim 1, characterized in that a metal oxide catalyst is used which has a composition corresponding to the general formula:

$Mo_{12}\ Fe_{0.5\text{-}6}\ Me^5_e\ Me^6_f\ Me^7_g\ Me^4_h\ Bi_i\ O_x$ IV

where
$Me^5$ is Ni and/or Co,
$Me^6$ is one or more of the elements P, B and Sb,
$Me^7$ is one or more of the elements K, Rb, Cs, Tl and In,
e is a number from 0 to 12,
f is a number from 0 to 6,
g is a number from 0.01 to 0.2,
h is a number from 0 to 3,
i is a number from 0 to 6, and
$Me^4$ and x have the meanings given in claim 1.

3. A process for the preparation of p-toluylaldehyde from p-xylene as claimed in claim 1, characterized in that a metal oxide catalyst is used which has a composition corresponding to the general formula:

$Mo_{12}W_{0.5\text{-}100}\ Me^8_k\ Me^9_l\ ,Me^{10}_m\ O_x$ V

where
$Me^8$ is one or more of the elements Bi, Fe, Ce, Mn, Cu and Zn,
$Me^9$ is one or more of the elements Sb, Cr, V and B,
$Me^{10}$ is one or more of the elements K, Rb, Cs, Tl and In,
k is a number from 0 to 10,
l is a number from 0 to 6,
m is a number from 0.01 to 1, and
x has the meaning given in claim 1.

**Revendications**

1. Procédé de préparation de benzaldéhydes substitués en position para, répondant à la formule

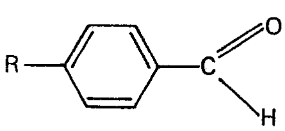

I,

dans laquelle R représente un groupe méthyle, méthoxy ou tert.-butyle par oxydation en phase vapeur d'un alkylbenzène répondant à la formule

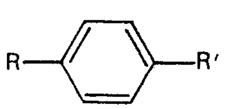

II,

dans laquelle R′ représente un radical alkyle, à l'aide d'oxygène ou de gaz contenant de l'oxygène en présence de catalyseurs contenant du molybdène, à des températures de 150 à 600°C, caractérisé en ce que l'on utilise un catalyseur à base d'oxydes métalliques dont la composition correspond à la formule générale

$Mo_{12}\ Me^1_a\ Me^2_b\ Me^3_c\ Me^4_d\ O_x$ III

dans laquelle
$Me^1$ représente un ou plusieurs des éléments Bi, Co, Ni, Fe, W, Nb, Ta,
$Me^2$ représente un ou plusieurs des éléments K, Rb, Cs, Tl, In,
$Me^3$ représente un ou plusieurs des éléments P, B, Sb, V, Sr, Cr,
$Me^4$ représente un ou plusieurs des éléments Mn, Re, Pd, Ir, Rh, Cu, Sn, Zn, Sm, Mg, Ce, Ag, Li, As, Ba, Ca,
a est un nombre allant de 0 à 24 mais dans le cas où le catalyseur contient W, un nombre de 0,5 à 110.
b est un nombre allant de 0,01 à 0,2 mais dans le cas où le catalyseur contient plus de 0,5 atome de Bi, V ou P, un nombre de 0,01 à 6,
c est un nombre allant de 0 à 6, mais dans le cas où a est égal à 0, un nombre de 0,5 à 5,
d est un nombre allant de 0 à 10 et
x est un nombre déduit de la somme des atomes de métaux et de phosphore présents et de leur stade d'oxydation le plus élevé.

2. Procédé selon la revendication 1 pour la préparation du p-tert.-butylbenzaldéhyde à partir du p-tert.-butyltoluène, caractérisé en ce que l'on utilise un catalyseur à base d'oxydes métalliques dont la composition correspond à la formule générale

$Mo_{12}\ Fe_{0,5\text{-}6}\ Me^5_e\ Me^6_f\ Me^7_g Me^4_h\ Bi_i\ O_x$ IV

dans laquelle
$Me^5$ représente Ni et/ou Co,
$Me^6$ représente un ou plusieurs des éléments P, B, Sb,
$Me^7$ représente un ou plusieurs des éléments K, Rb, Cs, Tl, In,
e est un nombre allant de 0 à 12,
f est un nombre allant de 0 à 6,
g est un nombre allant de 0,01 à 0,2,
h est un nombre allant de 0 à 3,
i est un nombre allant de 0 à 6 et
$Me^4$ et x ont les singifications indiquées dans la revendication 1.

3. Procédé selon la revendication 1 pour la préparation de l'aldéhyde p-toluique à partir du p-xylène, caractérisé en ce que l'on utilise un catalyseur à base d'oxydes métalliques dont la composition correspond à la formule générale

$Mo_{12}\ W_{0,5\text{-}100}\ Me^8_k\ Me^9_l,\ Me^{10}_m\ O_x$ V

dans laquelle
$Me^8$ représente un ou plusiers des éléments Bi, Fe, Ce, Mn, Cu, Zn,
$Me^9$ représente un ou plusieurs des éléments Sb, Cr, V, B,

$Me^{10}$ représente un ou plusieurs des éléments K, Rb, Cs, Tl, In,
k est un nombre allant de 0 à 10,
l est un nombre allant de 0 à 6,
m est un nombre allant de 0,01 à 1, et
x a la signification indiquée dans la revendication 1.